# EUROPEAN PATENT SPECIFICATION

(11) **EP 3 747 874 B1**
(45) Date of publication and mention of the grant of the patent: **01.03.2023**
(21) Application number: 19178098.0
(22) Date of filing: 04.06.2019
(51) Int. Cl.: C07D 255/02, A61B 1/00, C07D 275/06, C07D 417/12

(54) **SODIUM SACCHARIN HAVING CONJUGATED LIGAND, DERIVATIVES THEREOF AND PROCESS FOR THE PREPARATION THEREOF**
NATRIUM-SACCHARIN MIT KONJUGIERTEM LIGANDEN, DERIVATE DAVON UND VERFAHREN ZUR HERSTELLUNG DAVON
SACCHARINE DE SODIUM DOTÉE D'UN LIGAND CONJUGUÉ, SES DÉRIVÉS ET SON PROCÉDÉ DE PRÉPARATION

(43) Date of publication of application: 09.12.2020
(73) Proprietor: Kyung-in Synthetic Corporation, Incheon 22794 (KR)
(72) Inventor: KIM, Donggil, 63015 Jeju-si, Jeju-do (KR); LEE, Eui Jae, 14602 Bucheon-si, Gyeonggi-do (KR)
(74) Representative: Schiweck Weinzierl Koch Patentanwälte Partnerschaft mbB

(56) References cited:
- KR-A- 20170 089 245
- MAROUAN RAMI ET AL: "Carbonic anhydrase inhibitors: Gd(iii) complexes of DOTA- and TETA-sulfonamide conjugates targeting the tumor associated carbonic anhydrase isozymes IX and XII", NEW JOURNAL OF CHEMISTRY, vol. 34, no. 10, 1 January 2010 (2010-01-01), page 2139, XP55183743, ISSN: 1144-0546, DOI: 10.1039/c0nj00214c
- JANINA MOEKER ET AL: "Cyclic Secondary Sulfonamides: Unusually Good Inhibitors of Cancer-Related Carbonic Anhydrase Enzymes", JOURNAL OF MEDICINAL CHEMISTRY, vol. 57, no. 8, 24 April 2014 (2014-04-24) , pages 3522-3531, XP55149204, ISSN: 0022-2623, DOI: 10.1021/jm500255y

## Description

### BACKGROUND OF THE INVENTION

### 1. Field of the Invention

The present invention relates to sodium saccharin bound with a ligand, which is a substance having a selective targeting action on hypoxic tumors, and a process for preparation thereof.

### 2. Description of the Related Art

Among radio isotopes, a radio isotope [⁶⁸Ga] gallium emitting positron, principally can be obtained from extraction by the collapse according to physical half-life (270.95 days) of [⁶⁸Ge] germanium which is a parent nuclide, by a generator and it can be easily coordinated to a ligand having negative charge (Non-Patent Document 1). The radioisotope [⁶⁸Ga] gallium has long been used for diagnosis and treatment of tumors by marking biologically active molecules used for cancer cell targets.

About 90% of all currently known cancers are solid tumors. Concentrations of oxygen in solid tumors are very variable. Continued oxygen deficiency occurs necrosis, but viable tumor cells surround necrotic tissues and become hypoxic. Hypoxic tumor cells are characterized by their ability to resist glycolysis, angiogenesis, proliferation, metastasis, invasion and radiation therapy by altering their gene transcription patterns to overcome stress caused by oxygen deficiency.

Carbonic Anhydrase IX (CA IX) is present on the surface of hypoxic tumor cells. When cancer cells use glucose as an energy source in a state of oxygen deficiency, pyruvic acid is produced as a byproduct, which promotes conversion of aerobic respiration to anaerobic respiration. In this case, lactic acid accumulates, and therefore pH in the tumor rapidly decreases. Carbonic Anhydrase IX (CA IX) plays a large role in neutralizing carbon dioxide (CO₂) by using bicarbonate (HCO₃⁻) and maintaining the survival of cancer cells by regulating pH in the tumor cells (Non-Patent Document 2).

In addition, Carbonic Anhydrase IX (CA IX) is expressed only in hypoxic tumor cells, not normal cells, and saccharin (SAC) selectively binds to Carbonic Anhydrase IX without affecting surrounding normal cells, thereby inhibiting activity. Thus, studies on inhibition of proliferation of tumor cells due to such binding characteristics have been reported. A representative result of the study is an effective inhibition of cancer cell proliferation in the evaluation of anti-proliferative effect of saccharin on various cancer cell lines and MSCs by SW Kim Group of Korea University (Non-Patent Document 3).

The present invention is to develop a molecular imaging agent for tumor diagnosis using biological characteristics of tumor as described above.

### [Prior Art Document]

1. Velikyan I. 68Ga-Based Radiopharmaceuticals: Production and Application Relationship. Molecules Review. 2015. 12913-12943.
2. Mahon BP, Hendon AM, Driscoll JM, Rankin GM, Poulsen SA, Supuran CT, McKenna R. Saccharin: a lead compound for structure-based drug design of carbonic anhydrase IX inhibitors. 2015. 849-854.
3. JS Choi, Sang Yong Park, Man Gil Dong Beom Lee, Tae Bok Lee, Ji Hye Heo, Min Woo Lee, and Suhng Wook Kim. Estimation of Anti-proliferative Activity of Saccharin against Various Cancer Cell Lines and MSCs. 2016. 169-175.

### SUMMARY OF THE INVENTION

It is an object of the present invention to provide a compound having sodium saccharin (SAC) bound with a ligand which has high binding affinity for Carbonic Anhydrase IX (CA IX) in hypoxic tumors,

It is also an object of the present invention to provide a method for producing the above compound

It is also an object of the present invention to provide a contrast agent comprising the above compound

In order to solve the above problems, the present invention provides a compound having sodium saccharin (SAC) bound with a ligand compound,

According to one embodiment, the ligand compound may comprise 1,4,7-triazacyclononane-1,4,7-triacetic acid (NOTA).

According to one embodiment, the compound may comprise the structure as shown in formula 1.

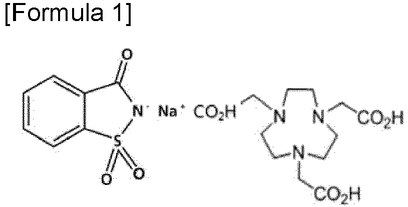

According to one embodiment, the compound may comprise the structure as shown in formula 2.

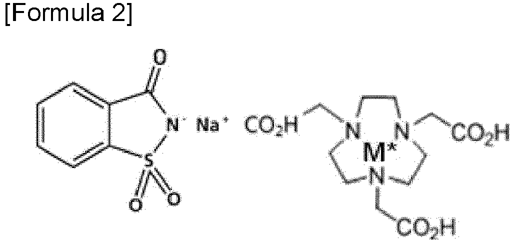

In the formula 2,
Sodium saccharin (SAC) is ionically bonded to 1,4,7-triazacyclononane-1,4,7-triacetic acid (NOTA) and M* is a radio isotope which is coordinated to the NOTA.

According to one embodiment, the radio isotope may be ⁶⁸Ga.

According to another embodiment of the present invention, there is provided a method for producing the above compound the method comprising the steps of:
ionically binding sodium saccharin (SAC) with a ligand compound to prepare a sodium saccharin-ligand compound, and
reacting the sodium saccharin-ligand compound with a radio isotope.

According to one embodiment, the ligand compound may comprise 1,4,7-triazacyclononane-1,4,7-triacetic acid (NOTA).

According to one embodiment, the radio isotope may be ⁶⁸Ga.

According to another embodiment of the present invention, there is provided a contrast agent comprising the above compound

In addition, the contrast agent can be used in Positron Emission Tomography (PET) for cancer diagnosis.

Other specific embodiments of the present invention are included in the following detailed description.

### EFFECT OF THE INVENTION

The present invention relates to sodium saccharin having conjugated ligand, and a process for preparation thereof. According to the present invention, there is provided a compound which is specifically bonded to hypoxic tumors, or derivatives thereof. Further, by applying the above compound to a contrast agent for cancer diagnosis, it is possible to effectively diagnose the tumor by improving the discrimination.

### BRIEF DESCRIPTION OF THE DRAWINGS

Fig. 1 is a graph showing the result of thin-layer chromatography (TLC) on a compound according to the present invention.
Fig. 2 is an image of positron emission computed tomography (PET/CT) using a compound according to the present invention.
Fig. 3 is a PET/CT image for identification of tumor selectivity.
Fig. 4 is a graph showing the distribution of a compound according to the present invention in tissues.

### DETAILED DESCRIPTION OF THE INVENTION

Since various modifications and variations can be made in the present invention, particular embodiments are illustrated in the drawings and will be described in detail in the detailed description. It should be understood, however, that the invention is not intended to be limited to the particular embodiments.

The invention is defined in the appended claims. In the following description of the present invention, detailed description of known functions will be omitted if it is determined that it may obscure the gist of the present invention.

Hereinafter, a compound or derivatives thereof according to an embodiment of the present invention will be described in more detail.

The present invention provides a compound having sodium saccharin (SAC) bound with a ligand compound,

According to one embodiment, the ligand compound may comprise 1,4,7-triazacyclononane-1,4,7-triacetic acid (NOTA). Such a metal affinity ligand compound can prevent radio isotopes from liberating in the body, thereby releasing the radioactive substance out of the body. From this, it can serve as a protecting agent by reducing cytotoxicity (Marouan Rami et al., Carbonic anhydrase inhibitors: Gd(III) complexes of DOTA- and TETA-sulfonamide conjugates targeting the tumor associated carbonic anhydrase isozymes IX and XII , New J. Chem., 2010, 34, 2139-2144; Silvio Aime et al., NMR relaxometric studies of Gd(III) complexes with heptadentate macrocyclic ligands, Magnetic Resonance in Chemistry (1998) Volume: 36, Issue: S1, Pages: S200-S208).

Specifically, the compound according to the present invention may comprise the compound of the following formula 1.

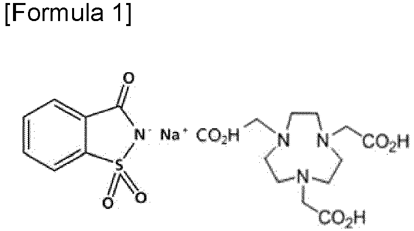

In the above formula 1, sodium of saccharin and a carboxyl group of 1,4,7-triazacyclononane-1,4,7-triacetic acid (NOTA) are bonded to each other.

According to one embodiment, the compound according to the present invention may comprise the structure of the following formula 2.

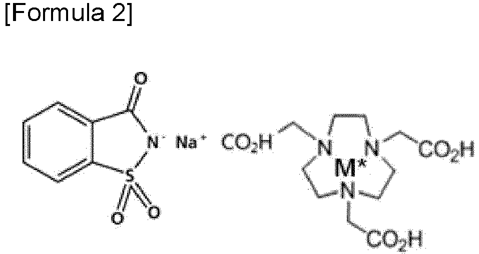

In the formula 2,
Sodium saccharin (SAC) is ionically bonded to 1,4,7-triazacyclononane-1,4,7-triacetic acid (NOTA), and
M* is a radio isotope which is coordinated to the NOTA.

According to one embodiment, the radio isotope may be ⁶⁸Ga.

The NOTA can coordinate with the radio isotope to easily form a complex.

That is, as described above, the present invention can provide a compound having conjugated ligand in order to bind a radio isotope to sodium saccharin (SAC) stably.

According to other embodiment of the present invention, there is provided a method for producing a compound the method comprising the steps of:
ionically binding sodium saccharin (SAC) with a ligand compound to prepare a sodium saccharin-ligand compound, and
reacting the sodium saccharin-ligand compound with a radio isotope.

According to one embodiment, the method of the present invention may comprise a step of reacting a solution of ⁶⁸Ga with a buffer solution of pH 5 to 6 and a compound of formula 1, for example, at 50 to 100 °C, for example, at 80 °C to form a compound of formula 2. The buffer solution may include, but is not limited to, for example, sodium acetate.

According to one embodiment, the ligand compound may comprise 1,4,7-triazacyclononane-1,4,7-triacetic acid (NOTA).

Specifically, the production process according to one embodiment of the present invention is shown in Scheme 1.

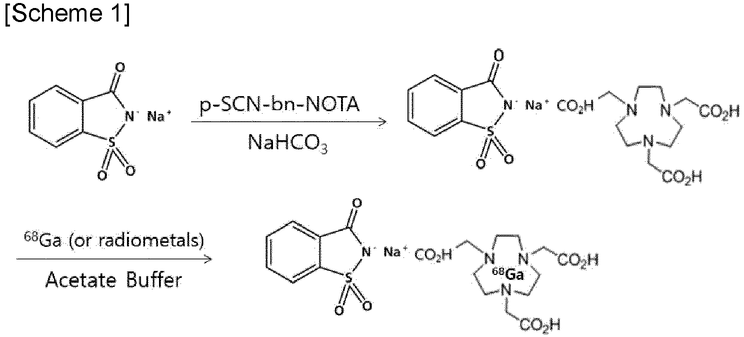

As shown in Scheme 1, in the present invention, a ligand was used to coordinate sodium saccharin with a metallic radio isotope. The ligand compound includes a compound in which sodium of saccharin are bonded to a carboxyl group of 1,4,7-triazacyclononane-1,4,7-triacetic acid (NOTA).

Sodium saccharin (SAC) has a property of specifically binding to carbonic anhydrase IX (CA IX), which is not expressed in normal cells but expressed only in hypoxic tumors. Sodium saccharin (SAC) is labeled with a radio isotope through the ligand compound. Due to such targeting to hypoxic tumors, the substance according to the present invention can be applied to a contrast agent.

Specifically, a contrast agent comprising a compound according to the present invention can be used as a contrast agent of Positron Emission Tomography (PET) for hypoxic tumors.

According to one embodiment, the PET contrast agent according to the present invention can be formulated to an injection or the like.

According to one embodiment, when the compound according to the present invention is used as a contrast agent, a non-toxic solution, which is an isotonic component with blood, may be contained as a diluent. The diluent may include, for example, sodium chloride solution, potassium chloride solution, sodium bicarbonate solution, Hartmann's solution, glucose solution, glucose physiological saline solution and the like. Specific examples of the diluent include phosphate buffer solution of pH 7.4 or the like.

Hereinafter, embodiments of the present invention will be described in detail so that those skilled in the art can easily carry out the present invention. The present invention may, however, be embodied in many different forms and should not be construed as limited to the embodiments set forth herein.

### Example 1: Preparation of Compound of Formula 1 (SAC-NOTA)

To prepare a compound of formula 1 (SAC-NOTA), a K₂CO₃ solution and 1 mg of a ligand compound, 1,4,7-triazacyclononane-1,4,7-triacetic acid (NOTA) were added to a reaction vessel and dissolved therein. In another vessel, 1.83 mg (5 eq.) of sodium saccharin (SAC) was dissolved in distilled water (D.W) and then added to the reaction vessel. After stirring at room temperature for 24 hours, separation and purification were carried out using a column (Pharmacia Fine Chemicals, Sephadex G-25).

### Example 2: Preparation of Compound of Formula 2 (SAC-NOTA-⁶⁸_{Ga})

To prepare a compound of formula 2 (SAC-NOTA-⁶⁸Ga), gallium was used as a radio isotope. ⁶⁸Ga which was extracted from a ⁶⁸Ga generator was obtained as a colorless transparent solution dissolved in a dilute hydrochloric acid solution. After taking it properly according to the amount of radioactivity required for the experiment (above 0.1mCi), the ⁶⁸Ga solution was placed in a suitable glass vessel and dried at 100 °C while blowing nitrogen therein. The dried ⁶⁸Ga is applied to the glass vessel in the form of a transparent film. 0.5 mL of the compound of formula 1 (SAC-NOTA) which was isolated and extracted in Example 1 was added to the reaction vessel coated with ⁶⁸Ga, and then the labeling reaction was allowed to proceed for 10 minutes at pH 5 to 6 at 80 °C. Reaction according to Example 2 is shown in Scheme 2.

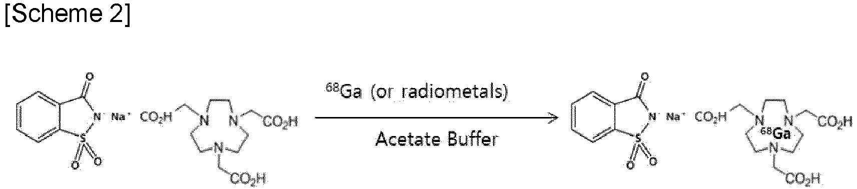

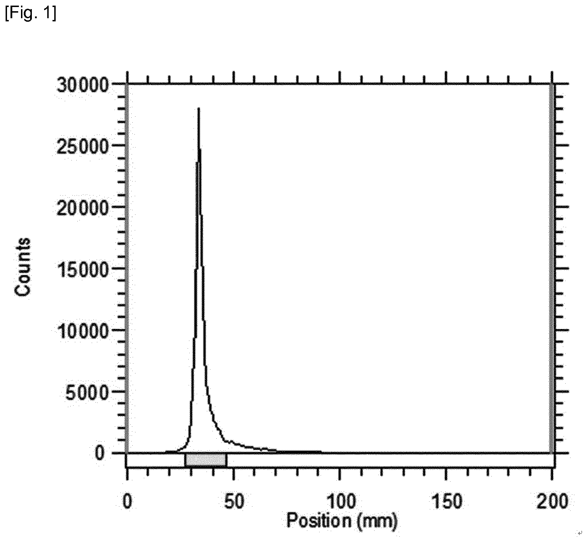

The final compound, the compound of formula 2 (SAC-NOTA-⁶⁸Ga), was adsorbed on a thin layer chromatography (TLC) plate and sufficiently developed with 0.1 M citrate buffer solution. From this, radiochemical yield and purity was identified. The results are shown in Fig. 1. Fig. 1 is a graph showing Radio-TLC (AR-2000, Eckert & Ziegler) results of the compound of formula 2 (SAC-NOTA-⁶⁸Ga).

As shown in Fig. 1, the radiochemical yield and purity from the TLC image are confirmed to be 98% or more on average.

### Experimental Example 1: Evaluation of Stability

In order to evaluate stability for the compound of formula 2 (SAC-NOTA-⁶⁸Ga) prepared in Example 2, stability in human serum and mouse serum was measured. 100 µL of 0.4 mCi of the compound of formula 2 (SAC-NOTA-⁶⁸Ga) was mixed with 0.5 mL of each of human serum and mouse serum, and then cultured at 37 °C. Thin layer chromatography (TLC) was performed using radiothin film chromatography (AR-2000, Eckert & Ziegler) equipment at the time to be measured (1 hour, 3 hours, 6 hours). The measurement results of stability of the compound of formula 2 (SAC-NOTA-⁶⁸Ga) in the serum over time are shown in Table 1 below.

**[Table 1]**

| Item | | 1 h | 3 h | 6 h |
|---|---|---|---|---|
| Compound of formula 2 | Human serum | 100% | 100% | 98% |
| | Mouse serum | 100% | 98.26% | 98.50% |

As shown in Table 1, since the form of the compound of formula 2 as a target compound is maintained, that is, the purity is maintained, it can be confirmed that the compound of formula 2 is stable for 6 hours or longer in human serum and mouse serum.

### Experimental Example 2: PET Image Acquisition of SAC-NOTA-⁶⁸Ga

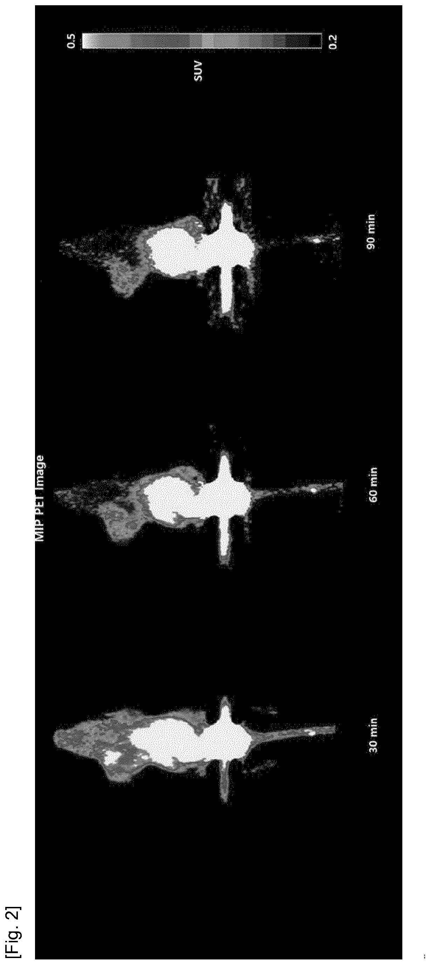

Positron emission tomography (PET) images were taken with PET/CT equipment for small animals (INVEON, Simens Medical Solutions). U87MG cells, a malignant glioma cell line, were implanted subcutaneously in the left shoulder of a nude mouse to form a tumor model. PET/CT images were obtained at 30 minutes, 60 minutes, and 90 minutes by injecting 0.23 mCi of the compound of formula 2 (SAC-NOTA-⁶⁸Ga) prepared in Example 2 into the tail vein of the tumor-transplanted mouse (nu/nu nude, 20 g). The results were shown in Fig. 2. As shown in Fig. 2, it can be observed that the signal increases at the tumor site after administration of the compound of formula 2 (SAC-NOTA-⁶⁸Ga).

### Experimental Example 3: Identification of Tumor Selectivity of SAC-NOTA-⁶⁸Ga

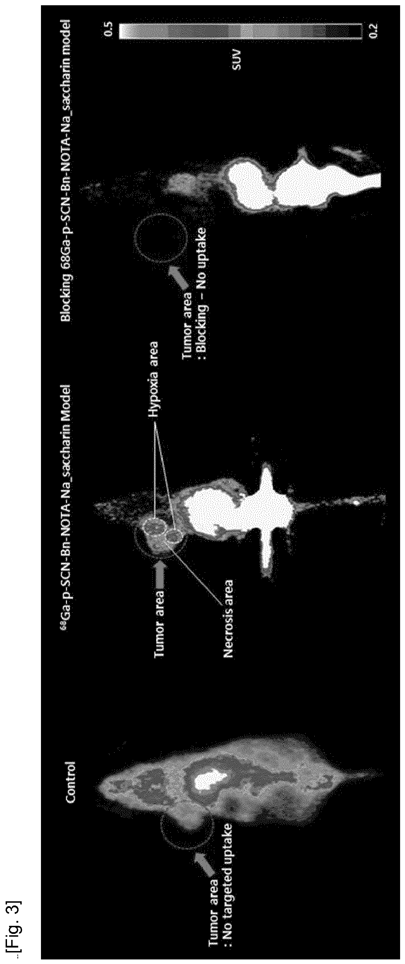

Experiments were carried out to identify selective specificity for receptors expressed in tumor cells of SAC-NOTA-⁶⁸Ga prepared in Example 2. U87MG cells, a malignant glioma cell line, were injected subcutaneously into the shoulder of a nude mouse to form a tumor model. First, sodium saccharin (10 mg/kg) was injected through the tail vein of the anesthetized mouse to block the receptor. After 30 minutes, 0.2 mCi of the SAC-NOTA-⁶⁸Ga was injected to acquire PET/CT images in the same manner as in Experimental Example 2. The results were shown in Fig. 3.

In Fig. 3, a PET/CT image (center) is taken by injecting SAC-NOTA-⁶⁸Ga without blocking the receptor present in the tumor and a PET/CT image (right) is taken by injecting SAC-NOTA-⁶⁸Ga after blocking the receptor present in the tumor by injecting sodium saccharin.

According to Fig. 3, it can be seen that the signal of the tumor site where the receptor is blocked is lower than the signal of the tumor site where the receptor is not blocked. From these results, it can be seen that SAC-NOTA-⁶⁸Ga has selective specificity for receptors expressed in the tumor cell.

### Experimental Example 4: Identification of Distribution of SAC-NOTA-^{s8}Ga in Tissues

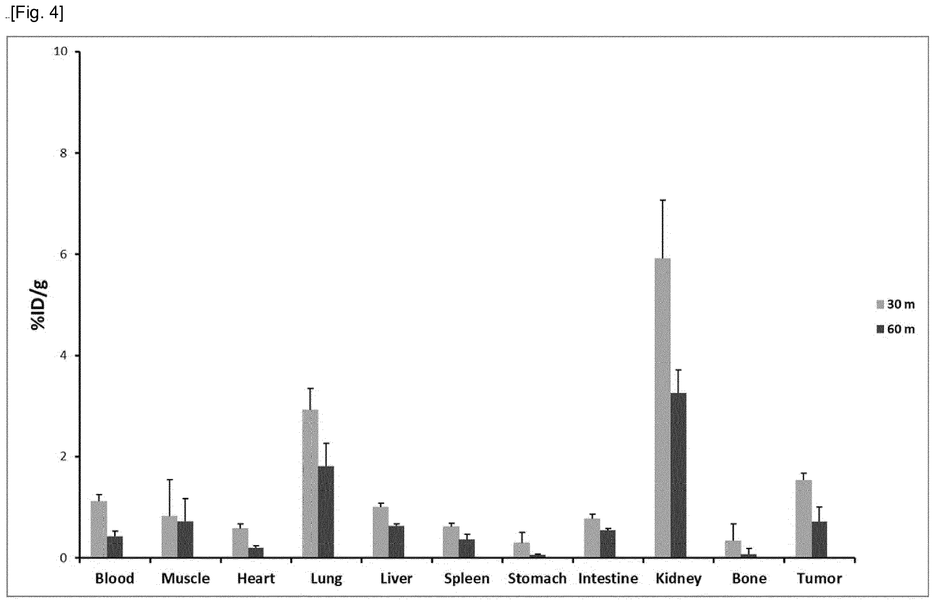

The distribution in mouse tissues of SAC-NOTA-⁶⁸Ga prepared in Example 2 was confirmed. U87MG cells, a malignant glioma cell line, were injected subcutaneously into the shoulder of a nude mouse (body weight 20 g) to form a tumor model. 0.01 mCi of SAC-NOTA-⁶⁸Ga was injected through the tail vein of the tumor-bearing mouse (n = 4). After 30 minutes and 60 minutes of injection, the organs (blood, muscle, heart, lung, liver, spleen, stomach, intestine, kidney, bone, tumor) were extracted, respectively. The radioactivity of the tissue was measured with a gamma counter. The results were shown in Fig. 4.

Fig. 4 is a graph showing a ratio of the tissue radioactivity to the amount of injection (% ID/g) measured in various organs after injection of SAC-NOTA-⁶⁸Ga over time.

From Fig. 4, it can be seen that the radioactivity in the tumor is maintained at 1.52% after 30 minutes of injection and at 0.69% after 60 minutes of injection. In addition, it can be seen that the radioactivity value of the liver tissue was kept low at 0.99% after 30 minutes and at 0.63% after 60 minutes of injection.

The above description is only illustrative of the technical idea of the present invention.

It should be noted that the embodiments disclosed in the present invention are intended to illustrate rather than limit the scope of the present invention. The scope of the present invention is not limited by these embodiments. The scope of protection of the present invention should be construed according to the following claims. The invention is defined in the appended claims.

## Claims

1. A compound having sodium saccharin (SAC) bound with a ligand compound.

2. The compound according to claim 1, wherein the ligand comprises 1,4,7-triazacyclononane-1,4,7-triacetic acid (NOTA).

3. The compound according to claim 1, wherein the compound comprises the structure of the following formula 1:

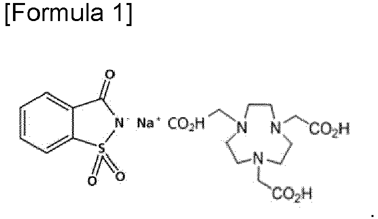

4. The compound according to claim 3, wherein the compound comprises the structure of the following formula 2: 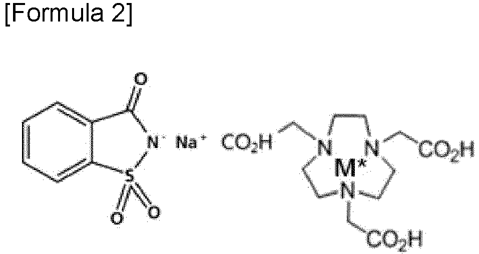 in the formula 2,
Sodium saccharin (SAC) is ionically bonded to 1,4,7-triazacyclononane-1,4,7-triacetic acid (NOTA) and
M* is a radio isotope which is coordinated to the NOTA.

5. The compound according to claim 4, wherein the radio isotope is ⁶⁸Ga.

6. A method for producing a compound, the method comprising the steps of:
ionically binding sodium saccharin (SAC) with a ligand compound to prepare a sodium saccharin-ligand compound, and
reacting the sodium saccharin-ligand compound with a radio isotope.

7. The method for producing a compound according to claim 6, wherein the ligand compound comprises 1,4,7-triazacyclononane-1,4,7-triacetic acid (NOTA).

8. The method for producing a compound according to claim 6, wherein the radio isotope is ⁶⁸Ga.

9. A contrast agent comprising the compound according to any of claims 1 to 5.

10. The contrast agent as defined in claim 9 for use in cancer diagnosis.

11. The contrast agent for the use of claim 10, wherein the contrast agent is used in Positron Emission Tomography (PET).

## Patentansprüche

1. Verbindung, bei der Natriumsaccharin (SAC) an eine Ligandenverbindung gebunden ist.

2. Verbindung nach Anspruch 1, wobei der Ligand 1,4,7-Triazacyclononan-1,4,7-triessigsäure (NOTA) umfasst.

3. Verbindung nach Anspruch 1, wobei die Verbindung die Struktur der folgenden Formel 1 umfasst:

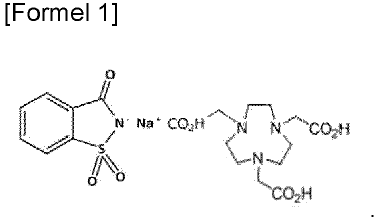

4. Verbindung nach Anspruch 3, wobei die Verbindung die Struktur der folgenden Formel 2 umfasst: 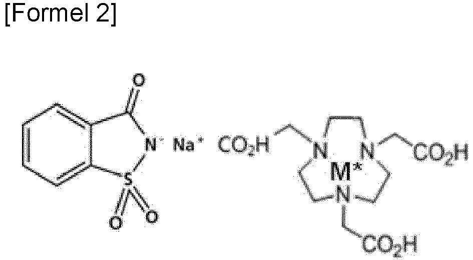 wobei in der Formel 2
Natriumsaccharin (SAC) ionisch an 1,4,7-Triazacyclononan-1,4,7-triessigsäure (NOTA) gebunden ist und
M* ein Radioisotop ist, das an NOTA koordiniert ist.

5. Verbindung nach Anspruch 4, wobei das Radioisotop ⁶⁸Ga ist.

6. Verfahren zum Herstellen einer Verbindung, wobei das Verfahren die Schritte umfasst:
ionisches Binden von Natriumsaccharin (SAC) an eine Ligandenverbindung, um eine Natriumsaccharin-Ligand-Verbindung herzustellen, und
Umsetzen der Natriumsaccharin-Ligand-Verbindung mit einem Radioisotop.

7. Verfahren zum Herstellen einer Verbindung nach Anspruch 6, wobei die Ligandenverbindung 1,4,7-Triazacyclononan-1,4,7-triessigsäure (NOTA) umfasst.

8. Verfahren zum Herstellen einer Verbindung nach Anspruch 6, wobei das Radioisotop ⁶⁸Ga ist.

9. Kontrastmittel, umfassend die Verbindung nach einem der Ansprüche 1 bis 5.

10. Kontrastmittel nach Anspruch 9 zur Verwendung bei der Krebsdiagnose.

11. Kontrastmittel zur Verwendung nach Anspruch 10, wobei das Kontrastmittel bei der Positronenemissionstomographie (PET) verwendet wird.

## Revendications

1. Composé ayant de la saccharine de sodium (SAC) liée à un composé ligand.

2. Composé selon la revendication 1, dans lequel le ligand comprend l'acide 1,4,7-triazacyclononane-1,4,7-triacétique (NOTA).

3. Composé selon la revendication 1, dans lequel le composé comprend la structure de la formule 1 suivante :

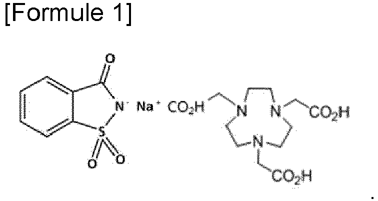

4. Composé selon la revendication 3, dans lequel le composé comprend la structure de la formule 2 suivante : 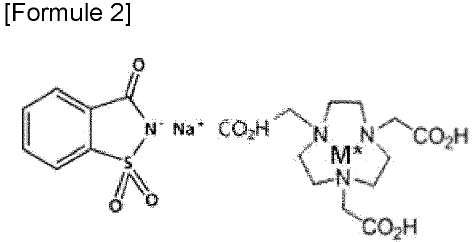 dans la formule 2,
la saccharine de sodium (SAC) est liée ioniquement à l'acide 1,4,7-triazacyclononane-1,4,7-triacétique (NOTA) et
M* est un radio-isotope qui est coordonné au NOTA.

5. Composé selon la revendication 4, dans lequel le radio-isotope est le ⁶⁸Ga.

6. Procédé de production d'un composé, le procédé comprenant les étapes suivantes :
la liaison ionique de saccharine de sodium (SAC) à un composé ligand pour préparer un composé saccharine de sodium-ligand, et
la mise en réaction du composé saccharine de sodium-ligand avec un radio-isotope.

7. Procédé de production d'un composé selon la revendication 6, dans lequel le composé ligand comprend l'acide 1,4,7-triazacyclononane-1,4,7-triacétique (NOTA).

8. Procédé de production d'un composé selon la revendication 6, dans lequel le radio-isotope est le ⁶⁸Ga.

9. Agent de contraste comprenant le composé selon l'une quelconque des revendications 1 à 5.

10. Agent de contraste tel que défini dans la revendication 9 pour utilisation dans le diagnostic du cancer.

11. Agent de contraste pour utilisation selon la revendication 10, dans lequel l'agent de contraste est utilisé en tomographie par émission de positons (TEP).
